# EUROPEAN PATENT APPLICATION

(11) **EP 4 575 503 A1**
(43) Date of publication of application: **25.06.2025**
(21) Application number: 24221077.1
(22) Date of filing: 18.12.2024
(51) Int. Cl.: G01N 33/543, B01L 3/00

(54) **CAPTURING AN ANALYTE HAVING AN AFFINITY MARKER**

(30) Priority: 22.12.2023 EP 23219877
(71) Applicant: Imec VZW, 3001 Leuven (BE)
(72) Inventor: DALBEN MADEIRA CAMPOS, Camila, 3001 Heverlee (BE); VOS, Rita, 3128 Baal (BE); ZHANG, Lei, 3010 Kessel-Lo (BE); HOELZ, Kathrin, 3001 Leuven (BE); LIU, Chengxun, 3001 Heverlee (BE)
(74) Representative: Winger

(57) **Abstract**

In a first aspect, the present invention relates to a device (10) for capturing an analyte having an affinity marker, comprising: i) a first electrode (31), ii) a second electrode (32); iii) a porous material (50) between the first (31) and second (32) electrode, iv) a first flow channel (41) for flowing a fluid medium in between the first electrode (31) and the porous material (50), and v) a second flow (42) channel for flowing a fluid medium in between the second electrode (32) and the porous material (50); wherein the first (31) and second (32) electrode are for generating an electrophoretic force steering-in operation-the analyte from the first flow channel (41) into the porous material (50), and the porous material (50) is a porous monolith of titania, silica or titania-silica comprising capture sites for binding to the affinity marker, having a thickness of at least 0.5 cm, having a contact area of at least 5 cm by 5 cm with each of the first and second flow channels, having a total surface area which is between 150 times to 200 times larger than a surface area of a footprint of the porous material, and having a pore size of at least 100 nm.

## Description

### Technical field of the invention

The present invention relates to separating an analyte (e.g. a biomolecule) having an affinity marker in a fluid medium, and more in particular to capturing said analyte in the fluid medium.

### Background of the invention

Separating an analyte from a (complex) sample is a fundamental process in various scientific and clinical applications, ranging from analytical (bio)chemistry to pharmaceutical research and diagnostics. In particular, the separation mRNA has seen growing interest in recent years, as a critical step towards the development and production of mRNA-based vaccines and similar treatments. Such applications hinge on the ability to isolate the analyte efficiently and rapidly.

Traditional approaches, however, are time-consuming, labour-intensive and require devices with a relatively large footprint. They therefore face limitations when dealing with high-throughput processing of large sample volumes within a constrained timeframe and physical space. In many cases, existing methods are not capable of meeting the demands of isolating substantial amounts of the analyte from solutions with volumes in the order of tens of litres within a processing time in the order of hours. This is also apparent from the review by Huang et al. (HUANG, Shengyun, et al. Advances in capillary electrophoretically mediated microanalysis for on-line enzymatic and derivatization reactions. Electrophoresis, 2018, 39.1: 97-110.), which summarized recent developments, applications, and innovations of capillary electrophoretically mediated microanalysis methods. Their review indicates that the recent efforts have been mainly directed towards analytical applications using small sample volumes.

Satterfield et al. (SATTERFIELD, Brent C., et al. Microfluidic purification and preconcentration of mRNA by flow-through polymeric monolith. Analytical chemistry, 2007, 79.16: 6230-6235.) disclosed the trapping and concentration of eukaryotic mRNA on UV-initiated methacrylate-based porous polymer monoliths functionalized with oligodeoxythymidines (oligo-dT's). But as it uses pressure driven flow only, there is a limit on the throughput that can be reached before the pressure drop becomes prohibitive. This happens because the small pore sizes increase the pressure drop, which increase is directly proportional to the thickness of the material and consequently to the surface area that can be achieved.

There is thus still a need in the art for techniques to separate an analyte in a (complex) sample which address at least some of the problems outlined above.

### Summary of the invention

It is an object of the present invention to provide good devices for capturing an analyte. It is a further object of the present invention to provide good arrangements and methods associated therewith. This objective is accomplished by devices, arrangements and methods according to the present invention.

It is an advantage of embodiments of the present invention that a relatively high sample volume can processed in a high-throughput manner.

It is an advantage of embodiments of the present invention that the combination of electrophoresis with a fluid flow through the flow channels increase the sample volume which can be processed in a given timeframe. It is a further advantage of embodiments of the present invention that the fluid flow helps evacuate chemical species (e.g. gas and/or H+/OH-) formed near the electrodes. It is yet a further advantage of embodiments of the present invention that the fluid flow prevents the analyte and/or contaminants from adsorbing to and/or reacting with the electrodes.

It is an advantage of embodiments of the present invention that the analyte can be quickly, effectively and efficiently captured in the fluid medium, even in a complex sample.

It is an advantage of embodiments of the present invention that the capture sites can selectively bind the analyte with respect to a contaminant. It is a further advantage of embodiments of the present invention that binding the analyte selectively allows to separate it very efficiently from the contaminant.

It is an advantage of embodiments of the present invention that through matching affinity markers with appropriate binding sites, different (biomolecular) analytes can be targeted, including in particular nucleotides such as mRNA.

It is an advantage of embodiments of the present invention that the analyte can subsequently be released for further processing.

It is an advantage of embodiments of the present invention that multiple devices can be easily linked together to realize more involved capture/separation actions.

It is an advantage of embodiments of the present invention that the device can be easily incorporated into existing fluidic systems.

It is an advantage of embodiments of the present invention that it can be realized in a relatively straightforward and economical fashion.

It is an advantage of embodiments of the present invention that it can be realized using materials and fabrication techniques that are fairly easily accessible.

In a first aspect, the present invention relates to a device for capturing an analyte having an affinity marker, comprising: i) a first electrode, ii) a second electrode; iii) a porous material between the first and second electrode, iv) a first flow channel for flowing a fluid medium in between the first electrode and the porous material, and v) a second flow channel for flowing a fluid medium in between the second electrode and the porous material; wherein the first and second electrode are for generating an electrophoretic force steering-in operation-the analyte from the first flow channel into the porous material, and the porous material is a porous monolith of titania, silica or titania-silica comprising capture sites for binding to the affinity marker, having a thickness of at least 0.5 cm, having a contact area of at least 5 cm by 5 cm with each of the first and second flow channels, having a total surface area which is between 150 times to 200 times larger than a surface area of a footprint of the porous material, and having a pore size of at least 100 nm.

In a second aspect, the present invention relates to an arrangement comprising a plurality of the devices as described in any embodiment of the first aspect

In a third aspect, the present invention relates to a method for capturing an analyte having an affinity marker using a device or arrangement as defined in any embodiment of the first or second aspect, comprising: a) providing the analyte in a fluid medium flowing through the first flow channel; b) operating the first and second electrode to generate a force field which steers the analyte from the first flow channel into the porous material; and c) capturing the analyte by letting the affinity marker bind to the capture sites.

Particular and preferred aspects of the invention are set out in the accompanying independent and dependent claims. Features from the dependent claims may be combined with features of the independent claims and with features of other dependent claims as appropriate and not merely as explicitly set out in the claims.

Although there has been constant improvement, change and evolution of devices in this field, the present concepts are believed to represent substantial new and novel improvements, including departures from prior practices, resulting in the provision of more efficient, stable and reliable devices of this nature.

The above and other characteristics, features and advantages of the present invention will become apparent from the following detailed description, taken in conjunction with the accompanying drawings, which illustrate, by way of example, the principles of the invention. This description is given for the sake of example only, without limiting the scope of the invention. The reference figures quoted below refer to the attached drawings.

### Brief description of the drawings

FIG 1 schematically depicts an illustrative cross-section of a device in accordance with embodiments of the present invention.
FIG 2 schematically shows a 3D view of an illustrative architecture for a device in accordance with embodiments of the present invention.

In the different figures, the same reference signs refer to the same or analogous elements.

### Description of illustrative embodiments

The present invention will be described with respect to particular embodiments and with reference to certain drawings but the invention is not limited thereto but only by the claims. The drawings described are only schematic and are non-limiting. In the drawings, the size of some of the elements may be exaggerated and not drawn on scale for illustrative purposes. The dimensions and the relative dimensions do not correspond to actual reductions to practice of the invention.

Furthermore, the terms first, second, third and the like in the description and in the claims, are used for distinguishing between similar elements and not necessarily for describing a sequence, either temporally, spatially, in ranking or in any other manner. It is to be understood that the terms so used are interchangeable under appropriate circumstances and that the embodiments of the invention described herein are capable of operation in other sequences than described or illustrated herein.

Moreover, the terms top, bottom, over, under and the like in the description and the claims are used for descriptive purposes and not necessarily for describing relative positions. It is to be understood that the terms so used are interchangeable with their antonyms under appropriate circumstances and that the embodiments of the invention described herein are capable of operation in other orientations than described or illustrated herein.

It is to be noticed that the term "comprising", used in the claims, should not be interpreted as being restricted to the means listed thereafter; it does not exclude other elements or steps. It is thus to be interpreted as specifying the presence of the stated features, integers, steps or components as referred to, but does not preclude the presence or addition of one or more other features, integers, steps or components, or groups thereof. The term "comprising" therefore covers the situation where only the stated features are present and the situation where these features and one or more other features are present. Thus, the scope of the expression "a device comprising means A and B" should not be interpreted as being limited to devices consisting only of components A and B. It means that with respect to the present invention, the only relevant components of the device are A and B.

Similarly, it is to be noticed that the term "coupled", also used in the claims, should not be interpreted as being restricted to direct connections only. The terms "coupled" and "connected", along with their derivatives, may be used. It should be understood that these terms are not intended as synonyms for each other. Thus, the scope of the expression "a device A coupled to a device B" should not be limited to devices or systems wherein an output of device A is directly connected to an input of device B. It means that there exists a path between an output of A and an input of B which may be a path including other devices or means. "Coupled" may mean that two or more elements are either in direct physical or electrical contact, or that two or more elements are not in direct contact with each other but yet still co-operate or interact with each other.

Reference throughout this specification to "one embodiment" or "an embodiment" means that a particular feature, structure or characteristic described in connection with the embodiment is included in at least one embodiment of the present invention. Thus, appearances of the phrases "in one embodiment" or "in an embodiment" in various places throughout this specification are not necessarily all referring to the same embodiment, but may. Furthermore, the particular features, structures or characteristics may be combined in any suitable manner, as would be apparent to one of ordinary skill in the art from this disclosure, in one or more embodiments.

Similarly, it should be appreciated that in the description of exemplary embodiments of the invention, various features of the invention are sometimes grouped together in a single embodiment, figure, or description thereof for the purpose of streamlining the disclosure and aiding in the understanding of one or more of the various inventive aspects. This method of disclosure, however, is not to be interpreted as reflecting an intention that the claimed invention requires more features than are expressly recited in each claim. Rather, as the following claims reflect, inventive aspects lie in less than all features of a single foregoing disclosed embodiment. Thus, the claims following the detailed description are hereby expressly incorporated into this detailed description, with each claim standing on its own as a separate embodiment of this invention.

Furthermore, while some embodiments described herein include some but not other features included in other embodiments, combinations of features of different embodiments are meant to be within the scope of the invention, and form different embodiments, as would be understood by those in the art. For example, in the following claims, any of the claimed embodiments can be used in any combination.

Furthermore, some of the embodiments are described herein as a method or combination of elements of a method that can be implemented by a processor of a computer system or by other means of carrying out the function. Thus, a processor with the necessary instructions for carrying out such a method or element of a method forms a means for carrying out the method or element of a method. Furthermore, an element described herein of an apparatus embodiment is an example of a means for carrying out the function performed by the element for the purpose of carrying out the invention.

In the description provided herein, numerous specific details are set forth. However, it is understood that embodiments of the invention may be practised without these specific details. In other instances, well-known methods, structures and techniques have not been shown in detail in order not to obscure an understanding of this description.

The following terms are provided solely to aid in the understanding of the invention.

As used herein, and unless otherwise specified, when reference is made to an electrode 'respective with' a flow channel-or vice versa-it is meant that the first electrode is respective with the first flow channel and the second electrode is respective with the second flow channel.

As used herein, and unless otherwise specified, the footprint of a structure (e.g. a porous material) can generally be defined as the area of an orthogonal projection of the structure onto a plane. This plane may for example be a surface of a substrate (cf. infra) facing the structure (e.g. porous material). If no preferred plane can be selected, the footprint may be defined as the area of the largest (i.e. yielding the highest area) orthogonal projection of the structure onto a plane one can make. Thus, the footprint will generally correspond to the area defined by the outer circumference of a planar cut through the structure. This planar cut may preferably be parallel to one or more substrates (cf. infra), but may also be at an angle (i.e. different from 0°) to any or all substrates.

In a first aspect, the present invention relates to a device for capturing an analyte having an affinity marker, comprising: i) a first electrode, ii) a second electrode; iii) a porous material between the first and second electrode, iv) a first flow channel for flowing a fluid medium in between the first electrode and the porous material, and v) a second flow channel for flowing a fluid medium in between the second electrode and the porous material; wherein the first and second electrode are for generating an electrophoretic force steering-in operation-the analyte from the first flow channel into the porous material, and the porous material is a porous monolith of titania, silica (Si) or titania-silica comprising capture sites for binding to the affinity marker, having a thickness of at least 0.5 cm, having a contact area of at least 5 cm by 5 cm with each of the first and second flow channels, having a total surface area which is between 150 times to 200 times larger than a surface area of a footprint of the porous material, and having a pore size of at least 100 nm.

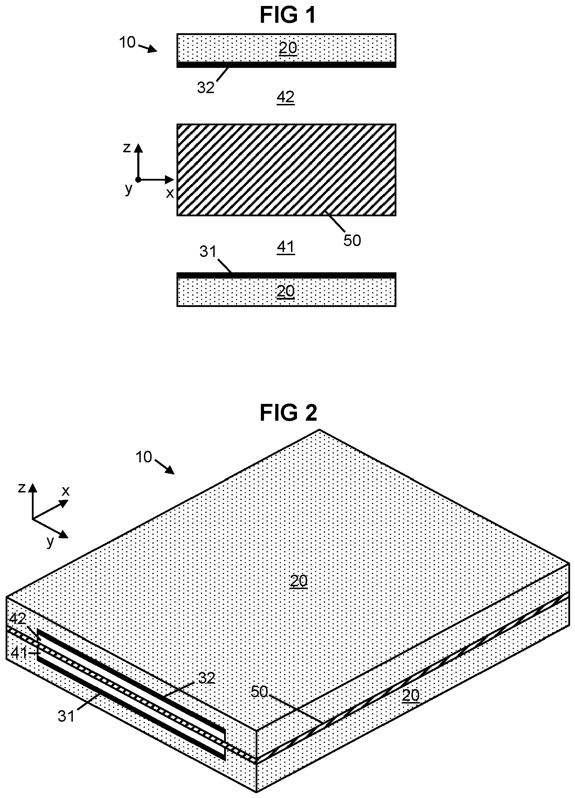

Such a device 10 may for instance have a cross-section as depicted in FIG 1, showing a first electrode 31, a first flow channel 41, a porous material 50, a second flow channel 42 and a second electrode 32.

The first and second electrode are for generating an electric field suitable for exerting an electrophoretic force on the analyte such that the analyte is (re)directed (i.e. steered) from the first flow channel into the porous material. They are typically not particularly limited by their structure and/or make-up, other than what is needed-as is well-known in the art-to achieve the aforementioned function. For example, the electrodes typically need to be in electrical contact with (e.g. directly exposed to) the flow medium in order to generate the required electric field across the flow channels and porous material. The aforementioned notwithstanding, the electrodes may for example be made of Pt or another material inert to the buffer. As depicted in FIG 1, the first and second electrode 31 and 32 are provided on/against an outer surface of substrate 20 and (at least partially) define an outer edge/boundary of the first and second flow channel 41 and 42, respectively. Notwithstanding, it is in general not required that the first and second electrode are provided on/against a substrate surface. Moreover, the first and second electrodes may-independently-also protrude/extend into the respective flow channel; so that the electrode is surrounded by the flow channel (e.g. the electrode is 'in' the respective flow channel).

The porous material is more specifically a porous monolith of titania (TiOₓ), silica (SiOₓ) or titania-silica (TiOₓ-SiOₓ). The porous material may for instance be a titania-silica porous monolith as disclosed by Yang et al. (YANG, Heqin, et al. Synthesis and characterization of hierarchical titania-silica monolith. Catalysis today, 2013, 216: 90-94.); which is incorporated herein by reference.

In embodiments, the porous material may have a pore size of between 100 nm and 1 mm, such as between 100 nm and 300 nm.

In embodiments, the porous material may be provided between substrates (e.g. between two substrates). For example, the porous material may be a (preformed) freestanding porous material that is assembled between the substrates. In particular embodiments, the porous material may be between the substrates. For example, the porous material may be suspended by being sandwiched at its edges between two substrates, and optionally additionally by being supported on protrusions (e.g. pillars) extending from the substrate. The substrate(s) may generally be any support structure(s) on/between which the porous material can be stably provided. In embodiments, the substrate(s) may be (independently) made of an amorphous or (poly)crystalline material, which may be an inorganic or organic material. They could be in the form of a slide, a sheet, a plate, a wafer, a casted or moulded product, etc. The substrate(s) may for example be (independently) selected from a glass (e.g. SiO₂) slide, a semiconductor (e.g. Si) wafer or a polymer sheet.

In embodiments, the first and second flow channel may each comprise an inlet and an outlet. In embodiments,-in operation-a fluid flow may flow through each flow channel. The fluid flow may typically be from the respective inlet to the respective outlet. In embodiments, the fluid flow may typically be substantially alongside (e.g. substantially parallel to) the porous material. The fluid flow in the first flow channel may generally be substantially independent from the fluid flow in the second flow channel. For example, even while both fluid flows may be substantially parallel to one another, they may nevertheless flow in opposite directions. Likewise, the flow velocity, fluid medium, dissolved/suspended species (e.g. ions), etc. need not be the same in both flow channels.

The first and second flow channel are generally at least present between the porous material and the respective electrode. Notwithstanding, the first and/or electrode may simultaneously be present 'in' (e.g. protruding into/be surrounded by) the respective flow channel. Typically, the first and second flow channel are preferably in direct physical contact with the porous material. Moreover, the first and second flow channel are preferably at least in electrical contact with the first and second electrode (thereby enabling the electrical connection to generate the required electric field; cf. supra). For example, the first and/or second flow channel may be in direct physical contact with the respective electrode. In embodiments, the first and/or second flow channel may be at least partially defined by (e.g. in) the substate (or by/in one or more of the substrates).

Compared to using only electrophoresis, the combination of electrophoresis with a fluid flow through flow channels as defined in the present invention has several advantages, namely: i) increase the sample volume which can be processed in a given time frame; ii) evacuate chemical species (e.g. gas and/or H⁺/OH⁻) formed near the electrodes; and iii) prevent the analyte and/or contaminants from adsorbing to and/or reacting with the electrodes.

In embodiments, any of the porous material, the first flow channel and the second flow channel may have a shortest dimension (e.g. a height) between 0.5 cm to 100 cm. In embodiments, any of the porous material, the first flow channel and the second flow channel may have a longest dimension (e.g. a length) between 5 cm to 100 cm.

The porous material comprising capture sites for binding the analyte generally entails that the capture sites are accessible to the analyte. Accordingly, the capture sites are typically (at least) present on a surface of the porous material. In embodiments, the capture sites may be for chemically and/or biochemically binding the analyte to the affinity marker. The affinity marker can for example include a member of a (bio)affinity pair (so that the affinity site corresponds to one half of the pair and the analyte comprises the complementary affinity marker) such as: antigen-antibodies, nucleic acid pair strands (e.g. DNA/DNA, DNA/RNA, poly-dT/poly-A), enzyme-substrate, metal-coordinating affinity tag-metal ion, etc.

In preferred embodiments, the capture sites may be for binding the analyte selectively with respect to a contaminant. This advantageously allows to separate the analyte very effectively from the contaminant.

The analyte may typically be any molecule which can be bound by a capture site through the affinity maker. Notwithstanding, it may in particular be a biomolecule such as an oligo- or polynucleotide (e.g. DNA or RNA, especially mRNA), an oligopeptide, a protein, etc.

In embodiments, the device may further comprise a condition generator for adjusting the binding conditions at the capture sites. The condition generator may for example comprise a radiant flux, temperature and/or pH controller (for adjusting the radiant flux-e.g. by UV light-, temperature and/or pH at the capture sites). Adjusting the binding conditions at the capture sites may for example be used to reverse the binding between the analyte and the capture site (thereby releasing the analyte), or to facilitate desorbing a further (e.g. non-specifically bound) molecule from the porous material; cf. infra.

In embodiments, the device may further comprise a flow generator (e.g. a pump) for flowing a fluid medium in the first and second flow channels.

In embodiments, the device may further comprise a control unit configured for instructing the first and second electrodes. Moreover, the control unit may be further configured for instructing the aforementioned flow generator and/or condition generator.

In embodiments, the device may further comprise a reservoir for collecting the released analyte. In embodiments, the reservoir may be fluidically coupled to the outlet of the second flow channel.

In embodiments, any feature of any embodiment of the first aspect may independently be as correspondingly described for any embodiment of any of the other aspects.

In a second aspect, the present invention relates to an arrangement comprising a plurality of the devices as described in any embodiment of the first aspect.

In embodiments, the plurality of the devices may be fluidically coupled in parallel and/or in series.

In embodiments, the arrangement may further comprise a flow generator (e.g. a pump) for flowing a fluid medium in the first and second flow channels.

In embodiments, the arrangement may further comprise a control unit configured for instructing the first and second electrodes. Moreover, the control unit may be further configured for instructing the aforementioned flow generator.

In embodiments, any feature of any embodiment of the second aspect may independently be as correspondingly described for any embodiment of any of the other aspects.

In a third aspect, the present invention relates to a method for capturing an analyte having an affinity marker using a device or arrangement as defined in any embodiment of the first or second aspect, comprising: a) providing the analyte in a fluid medium flowing through the first flow channel; b) operating the first and second electrode to generate a force field which steers the analyte from the first flow channel into the porous material; and c) capturing the analyte by letting the affinity marker bind to the capture sites.

In preferred embodiments, step c may performed selectively with respect to a contaminant. Accordingly, step c may comprise letting the contaminant pass through (or adsorb non-specifically to) the porous material.

In embodiments, the method may comprise a further step d-after step c-of: d) desorbing (e.g. by adjusting temperature and/or pH) a non-specifically bound contaminant from the porous material.

In embodiments, the method may comprise a further step e-after step d, if present-of: e) releasing the analyte by reversing (e.g. by adjusting temperature or using UV light) the binding between the affinity marker and the capture sites.

In embodiments, the method may further comprise collecting released unbound molecules and/or analytes (cf. supra).

In embodiments, the method may comprise further processing the analyte and/or the captured molecule (e.g. further processing the analyte). While the present invention generally deals with capturing an analyte (and thereby typically separating it from the fluid and/or contaminants in the fluid), this is not necessarily the end goal and the method can be readily extended towards various further processes.

In embodiments, any feature of any embodiment of the third aspect may independently be as correspondingly described for any embodiment of any of the other aspects.

The invention will now be described by a detailed description of several embodiments of the invention. It is clear that other embodiments of the invention can be configured according to the knowledge of the person skilled in the art without departing from the true technical teaching of the invention, the invention being limited only by the terms of the appended claims.

### Example

We now refer to FIG 2, showing an illustrative example of a device 10 in accordance with the present invention. The cross-section of this device 10 may also be as depicted in FIG 1.

The instant device 10 comprises a freestanding porous material 50-like a membrane-which is assembled between two substrates 20. The porous material 50 is thereby suspended by being sandwiched at its edges between two substrates 20. Optionally, small pillars may be provided (e.g. one or both of the substrates 20 may be outfitted therewith) under and/or above the porous material 50 to further support/sustain it. The freestanding porous material (e.g. membrane) 50 may preferably be made of a porous material, e.g. with a pore size of at least 100 nm. For example, the porous material may be a porous monolith of titania, silica or titania-silica (e.g. as described by Yang et al.; cf. supra). A treatment (e.g. a plasma or UV treatment) may be applied to the porous material to generate (additional) functional groups on the surface of the porous material. Depending on the application, these functional groups can be used directly as capture site or can be used to bind a capture probe thereto.

Each of the two substrates 20 are further provided with a cavity and an electrode 31 or 32. Accordingly,-after assembly- the porous material 50 is between the pair of electrodes 31 and 32, and two flow channels 41 and 42 are defined (respectively between the first electrode 31/second electrode 32 and the porous material 50).

The above device architecture is typically particularly suited towards larger-scale devices, e.g. for industrial applications. Typical dimensions for the porous material and each flow channel may for example be a height (along the z-direction) of in order of millimetres to about 0.5 cm, and a width (i.e. along the y-direction) and length (i.e. along the x-direction) of at least 5 cm (e.g. up to tens of centimetres). Moreover, the porous material has a total surface area between 150 to 200 times larger than its footprint.

In operation, a fluid flow is generated in each flow channel (from a respective inlet to the respective outlet-not depicted in FIG 2-; i.e. substantially alongside/parallel to the porous material 50). Simultaneously, the first electrode 31 and second electrode 32 are operated to generate a suitable electrophoretic field across the porous material 50. Upon providing the analyte in the fluid medium in the first flow channel 41, an electrophoretic force thereby acts on the analyte, steering it from the first flow channel 41 into the porous material 50. Inside the porous material 50, the analyte then (preferably selectively) interacts with-and is captured by-the captures sites, thereby separating it from the rest of the fluid species. More specifically, the fluid and other species (e.g. contaminants) which are not (sufficiently) affected by the electrophoretic field continue to flow through the first flow channel 41 and can be collected at the first outlet. On the other hand, the species (e.g. contaminants) which are steered into the porous material, but which do not bind with the capture sites flow through the porous material and can-through the second flow channel 42-be collected at the second outlet.

Depending on the specifics of the case (e.g. different materials involved, desired separation, etc.), it may be possible that a non-trivial amount of further species (i.e. other than the analyte) becomes (reversibly) adsorbed to the porous material 50 (to the captures sites as such or otherwise). However, the binding affinity of these species is generally lower than that of the analyte, so that it is possible to desorb them while keeping the analyte bound. To promote this, it can be useful to (slightly) vary the conditions (e.g. temperature and/or pH) in the device.

Finally, where the capture sites are selected such that the binding with the analyte is reversible, the analyte can be released when desired (depending on the capture site, based on temperature or using UV light) and collected at the second outlet.

It is to be understood that although preferred embodiments, specific constructions, configurations and materials have been discussed herein in order to illustrate the present invention. It will be apparent to those skilled in the art that various changes or modifications in form and detail may be made without departing from the scope of the invention as defined in the appended claims.

## Claims

1. A device (10) for capturing an analyte having an affinity marker, comprising:
i) a first electrode (31),
ii) a second electrode (32);
iii) a porous material (50) between the first (31) and second (32) electrode,
iv) a first flow channel (41) for flowing a fluid medium in between the first electrode (31) and the porous material (50), and
v) a second flow channel (42) for flowing a fluid medium in between the second electrode (32) and the porous material (50);
wherein
- the first (31) and second (32) electrode are for generating an electrophoretic force steering-in operation-the analyte from the first flow channel (41) into the porous material (50), and
- the porous material (50) is a porous monolith of titania, silica or titania-silica,
- comprising capture sites for binding to the affinity marker,
- having a thickness of at least 0.5 cm,
- having a contact area of at least 5 cm by 5 cm with each of the first (41) and second (42) flow channels,
- having a total surface area which is between 150 times to 200 times larger than a surface area of a footprint of the porous material (50), and
- having a pore size of at least 100 nm.

2. The device (10) according to claim 1, wherein the capture sites are for binding to the affinity marker selectively with respect to a contaminant.

3. The device (10) according to any of the previous claims, wherein the capture sites are for (bio)chemically binding to the affinity marker.

4. The device (10) according to any of the previous claims, further comprising a condition generator for adjusting the binding conditions at the capture sites.

5. The device (10) according to any of the previous claims further comprising:
- a flow generator for flowing a fluid medium in the first and second flow channels (42), and
- a control unit configured for instructing the flow generator and the first (31) and second (32) electrodes.

6. The device (10) according to any of the previous claims, further comprising a reservoir for collecting the released analyte.

7. An arrangement comprising a plurality of the devices (10) according to any of the previous claims.

8. The arrangement according to claim 8, wherein the plurality of the devices (10) are fluidically coupled in parallel and/or series.

9. The arrangement according to any of the claims 8 or 9 as depending on any of the claims 1 to 5, further comprising:
- a flow generator for flowing a fluid medium in the first and second flow channels (42) of the plurality of devices (10), and
- a control unit configured for instructing the flow generator and the first (31) and second (32) electrodes of the plurality of devices (10).

10. A method for capturing an analyte having an affinity marker using a device (10) or arrangement as defined in any of the previous claims, comprising:
a) providing the analyte in a fluid medium flowing through the first flow channel (41);
b) operating the first (31) and second (32) electrode to generate a force field which steers the analyte from the first flow channel (41) into the porous material (50); and
c) capturing the analyte by letting the affinity marker bind to the capture sites.

11. The method according to claim 11, comprising a further step d-after step c-of:
d) desorbing a non-specifically bound contaminant from the porous material (50).

12. The method according to any of claims 11 to 12, comprising a further step e-after step d, if present-of:
e) releasing the analyte by reversing the binding between the affinity marker and the capture sites.
